# EUROPÄISCHE PATENTSCHRIFT

(11) **EP 3 273 919 B1**
(45) Veröffentlichungstag und Bekanntmachung des Hinweises auf die Patenterteilung: **18.12.2019**
(21) Anmeldenummer: 16712024.5
(22) Anmeldetag: 23.03.2016
(51) Int. Cl.: A61F 2/945, A61F 2/958, A61F 2/844

(54) **ROHRFÖRMIGE HÜLSE UND SYSTEM ZUR ATRAUMATISCHEN BEHANDLUNG VON HOHLORGANEN**
TUBULAR SLEEVE AND SYSTEM FOR THE ATRAUMATIC TREATMENT OF HOLLOW ORGANS
GAINE TUBULAIRE ET SYSTÈME POUR LE TRAITEMENT SANS TRAUMATISME D'ORGANES CREUX

(30) Priorität: 23.03.2015 DE 102015104338
(43) Veröffentlichungstag der Anmeldung: 31.01.2018
(73) Patentinhaber: BVS - Best Vascular Solutions GmbH, 53173 Bonn (DE)
(72) Erfinder: BROHM-SCHMITZ-RODE, Andrea, 52070 Aachen (DE)
(74) Vertreter: HGF Europe LLP
(86) Internationale Anmeldenummer: PCT/EP2016/056429
(87) Internationale Veröffentlichungsnummer: WO 2016/151035

(56) Entgegenhaltungen:
- WO-A1-96/10967
- US-A- 5 507 770
- US-A- 6 059 823
- US-A1- 2010 249 946

## Beschreibung

Die vorliegende Erfindung betrifft eine rohrförmige Hülse und ein System zur atraumatischen Behandlung von Hohlorganen.

Zur Schienung von Engstellungen in menschlichen Hohlorganen, wie z.B. in Blutgefäßen, sind Stents bekannt. Ein Stent (Gefäßstütze) ist ein medizinisches Implantat, das in ein Hohlorgan einbringbar ist. Es handelt sich meist um ein rohrförmiges Gittergerüst aus Metall oder Kunststoff. Der Stent soll den betroffenen Abschnitt eines Hohlorganes stützen und auf diese Weise dauerhaft offen halten.

In der Regel werden Stents mittels eines Katheters oder eines Ballonkatheters zum Implantationsort befördert. Hierfür wird der Stent auf einem Ballonkatheter angeordnet. Der Stent soll dabei einen möglichst geringen Außendurchmesser aufweisen, um beim Einführen in den menschlichen und/oder tierischen Körper das entsprechende Hohlorgan möglichst wenig zu beschädigen. Hierfür wird meistens mittels Radialkräften auf den Stent eingewirkt, sodass eine konzentrische Durchmesserreduktion erfolgt. Ist der Stent am Implantationsort angeordnet, wird der Ballonkatheter inflatiert, sodass eine konzentrische Aufweitung des Stents erfolgt.

Das Stentmaterial kann jedoch in manchen Fällen eine Gerinnselbildung (Thrombogenität) auslösen. Weiterhin kann es durch die mechanische Beanspruchung bei der Inflation des Ballonkatheters zu einer Verletzung der Gefäßwandung des Hohlorganes kommen. Der Stent führt häufig langfristig zu einer chronischen Reizung. Auf diese Reizung reagiert die Gefäßwandung mit einer Überproduktion von Wandzellen und sogenannter extrazellulärer Matrix (Hyperplasie). Eine solche Gefäßwandproliferation kann derart stark ausgeprägt sein, dass es zu einer Wiederverengung des Blutgefäßes (Restenose) kommt. Aufgrund der Thrombogenität der Risse in der Gefäßwandung und auch des Stentmaterials wird häufig eine medikamentöse Antikoagulationsbehandlung durchgeführt, die eine Gerinnselbildung verhindern soll. Diese Therapie kann jedoch Nebenwirkungen haben. Daher wäre eine Reduktion der Medikation wünschenswert.

Weiterhin wird häufig versucht, die Gefäßwandproliferation durch sogenannte "Drug Eluting Stents" zu reduzieren. Derartige Stents sind in der Regel mit einem Polymer beschichtet, in das antiproliferative Wirkstoffe eingelagert sind bzw. sind diese Stents mit derartigen Wirkstoffen dotiert. Die Freisetzung der Wirkstoffe am Implantationsort reduziert die Überproduktion von Wandzellen. Jedoch verhindern diese Wirkstoffe bei manchen Patienten das Einwachsen des Stents in die Wandung des Gefäßes. Nach Beendigung der Antikoagulations-Medikation kann es zu sogenannten Spätthrombosen kommen, weil der Stent gar nicht oder nicht vollständig eingewachsen ist.

Um die vorstehenden Probleme zu vermeiden, werden neue Stentkonzepte verfolgt, zu denen auch sogenannte bioresorbierbare Stents zählen. Diese können aus biologisch abbaubaren Metalllegierungen, z. B. mit einem hohen Magnesiumanteil, oder aus biodegradierbaren Polymeren, z. B. Polylactid, ausgebildet sein. Hierbei ist vorgesehen, dass derartige Stents die Gefäßwandung einige Monate stützen und anschließend durch körpereigene Substanzen biologisch abgebaut werden. Auf diese Weise soll auch die mechanische Reizung auf die Gefäßwandung verringert werden und es soll zu weniger Restenose kommen. Ersten Studien zufolge kann aber auch bei biodegradierbaren Stents nicht auf die Beladung mit antiproliferativen Wirkstoffen verzichtet werden, weil die überschießende Gefäßwandreaktion bereits in den ersten Monaten nach der Implantation unterdrückt werden muss.

In der DE 10 2012 007 640 sind ein Stent und ein Stent-Implantationskatheter offenbart. Ein Stentkörper dieses Stents soll insbesondere eine aus einem expandierbaren Material ausgebildete Innenwandung derart aufweisen, dass nach der Implantation eine geschlossene Hülle ausgebildet wird. Die geschlossene Hülle soll dabei als eine Art Wundverschluss fungieren. Gemäß einer weiteren Ausführungsform soll der Stentkörper sowohl innenseitig als auch außenseitig eine Wandung aus expandierbarem Material aufweisen. Das Material ist hierbei direkt oder indirekt mit dem Stentkörper verbunden. Daher erfolgt die Expansion des Materials mit dem Stentkörper zusammen. Demgemäß werden der Stentkörper und die Wandungen mit einer konzentrischen Durchmesseraufweitung expandiert.

Bei der Stentimplantation sind Durchmesseraufweitungen mit dem Faktor 1:3 oder 1:4 üblich. Dies bedeutet eine Umfangzunahme um einen Faktor von 18 bis 24. Daher erfordern derartige mit dem Stent verbundene Wandungen eine hohe plastische Dehnbarkeit des Materials. Dies stellt jedoch materialtechnisch eine große Herausforderung dar.

Aus der WO 02 076 700 A1 geht ein Ballonkatheter hervor. Das den Ballonkatheter ausbildende Material ist hierbei plissiert und gefaltet, wobei das Material mit einem Katheterschaft fest verbunden ist.

In der US 5 443 495 A1 ist eine Angioplastie-Ballonimplantat-Vorrichtung offenbart. Hierbei ist vorgesehen, dass nach der Entfaltung eines Ballonkatheters ein Teil der Ballonwandung vom Ballonkatheter ablösbar ist. Dies soll durch die bei der Expansion des Ballonkatheters frei werdende Expansionskraft erfolgen, wobei Sollbruchstellen vorgesehen sind, um fest mit dem Katheterschaft verbundene Endsegmente der Ballonfolie abzutrennen. Die Ballonfolie ist hierbei integraler Bestandteil des Ballonkatheters. Weiterhin kann auch vorgesehen sein, auf dem Ballonkatheter einen Polymerstent anzuordnen.

Heute üblicherweise eingesetzte Stents weisen selbst bei besonders miniaturisierten Ausführungen, wie z.B. für Hirnarterien oder periphere Koronararterien, im expandierten Zustand einen Durchmesser von 1,5 mm bis 2,5 mm auf. Im expandierten Zustand haben die Gitteröffnungen, d.h. die maximalen Abstände zwischen den expandierten Stentstreben, eine Weite von mehr als 150µm.

Bei Stents, die mittels Ballonkatheter expandiert werden, erfolgt die Durchmesseraufweitung aufgrund einer plastischen Verformung der Stentstreben, insbesondere in den die Stentstreben verbindenden Knotenpunkten. Es findet somit eine plastische Verformung des Stentmaterials statt. Hierbei kommt es jedoch fast immer zu einem Zusammenziehen bzw. einem Zurückfedern (Recoil) des Stents, was zu einer ersten Wiederverengung des behandelten Hohlorganes führen kann. Für diese Rückstellungsneigung sind in der Regel Rückstellkräfte im Stentmaterial, wie z. B. Nitinol, verantwortlich.

In der DE 10 2006 020 687 A1 ist eine Stentgraft-Prothese offenbart. Diese Stentgraft-Prothese umfasst eine flexible Hülle zur Auskleidung eines Aneurysmas und kann einen der Hülle zugeordneten Aorteneinsatz in Form einer Gefäßprothese mit aufweitbaren Stützstrukturen aufweisen. Weiterhin können die Hülle und der Aorteneinsatz an ihren proximalen und distalen Enden derart abdichtend miteinander verbunden sein, dass eine Kammer ausgebildet wird. Die äußere Hülle ist derart überdimensioniert, dass Falten quer zur Längsrichtung der Hülle innerhalb des Aneurysmas ausgebildet sind.

Aus der US 2005 / 0 090 888 A1 geht eine plissierte Stentvorrichtung hervor. Der Stent ist im plissierten Zustand auf einem Ballon angeordnet, um eine plissierte Ballonbaugruppe auszubilden.

In der DE 2005 056 529 A1 ist eine komprimierbare Gewebestütze offenbart. Diese in etwa rohrförmige Gewebestütze ist aus einem Formgedächtnismaterial ausgebildet, wobei die Gewebestütze in ihrer Längsachse ein oder mehrfach gefaltet ist.

Aus der US 2002 / 0 045 930 A1 gehen eine Stentgraft-Entfaltungsvorrichtung sowie ein entsprechendes Verfahren hervor. Der Stentgraft selbst ist entlang seiner Längsachse gefaltet. Weiterhin kann der rohrförmige Stentgraft mittels Fasern verstärkt sein, die entweder in dem rohrförmigen Stentgraft eingebettet sind oder in einer separaten Schicht koaxial auf dem rohrförmigen Stentgraft aufgebracht sind.

Aus der US 2005/0 125 053 A1 geht eine rohrförmige medizinische Vorrichtung, wie zum Beispiel ein Stent oder ein Katheter, hervor. Dieser weist mehrere Reihen von Schlitzen auf, wobei die Vorrichtung auf einen kleineren Durchmesser zusammenfaltbar ausgebildet ist.

In der US 2008 / 0 262 594 A1 sind ein Stentgraft-Abdichtungssystem und ein entsprechendes Verfahren beschrieben. Dabei ist ein innerer rohrförmiger Körper vorgesehen, der um seine Längsachse plissiert ist, wobei auf dem inneren Körper ein Stent anordbar ist. Das rohrförmige Graftmaterial kann mit einem Abdichtungsstent an dessen äußeren Faltungen verbunden sein.

In der WO 96/10967 A1 sind ein intraluminaler Stent sowie ein Verfahren zu dessen Einbringung in den menschlichen Körper offenbart. Dieser rohrförmige Stent kann zum Einbringen in den menschlichen Körper um eine Längsachse der Vorrichutung gerichtete Faltungen aufweisen. Weiterhin ist der Stent aus einer Innenwandung und einer Außenwandung ausgebildet, die an ihren stirnseitigen Enden derart miteinander verbunden sind, dass im Bereich zwischen den Wandungen ein in etwa röhrenförmiges Kompartiment ausgebildet ist. Dieser Stent soll zur Behandlung eines Aortenaneurysmas oder von verletzten Blutgefäßen verwendbar sein.

Aus der US 5,507,770 A gehen ebenfalls ein intraluminaler Stent und ein entsprechen-des Verfahren zu dessen Implantation in einem Blutgefäß hervor. Dieser rohrförmige Stent umfasst eine Außenwandung und eine Innenwandung, zwischen denen eine Kammer ausgebildet ist. Weiterhin kann dieser Stent zum Einbringen in den menschlichen Körper eine Faltung aufweisen. In der US 6,059,823 A ist eine endovaskulare Vorrichtung zur perkutanen Behandlung von Aneurysmen in der Aorta und zur Behandlung von vaskularen Anomalien offenbart. Zum Einbringen in den menschlichen Körper kann diese Vorrichtung eine Faltung aufweisen. Weiterhin können an einer Mantelwandung der Vorrichtung rohrförmige Kanäle vorgesehen sein. Diese sind insbesondere zur Aufnahme von Drähten bei der Einbringung der Vorrichtung in den menschlichen Körper vorgesehen. Diese röhrenförmigen Abschnitte können sich sowohl in Längsrichtung als auch in Querrichtung der Vorrichtung erstrecken. Weiterhin begrenzen die röhrenförmigen Abschnitte einen Raum.

Aus der US 2010/249946 A1 geht ein transkutan einstellbares und mit einer Flüssigkeit gefülltes Implantat hervor. Dieses Implantat umfasst eine einzelne Wandung und ist nach dem Einbringen in den menschlichen Körper mittels einer Flüssigkeit befüllbar. Weiterhin kann dieses Implantat mit einem medizinischen Wirkstoff und/oder einer heilungsfördernden biologischen Substanz versehen sein. Die Wandung des Implantats kann auch aus zwei Wandungen ausgebildet sein, jedoch sind diese beiden Wandungen immer unmittelbar aneinander anliegend ausgebildet.

Aufgabe der vorliegenden Erfindung ist es, ein medizinisches Instrument zur atraumatischen Behandlung von Hohlorganen bereitzustellen, welches eine Alternative zu den aus dem Stand der Technik bekannten medizinischen Vorrichtungen, wie z.B. Stents, darstellt und breitere Anwendungsmöglichkeiten bietet.

Eine weitere Aufgabe der vorliegenden Erfindung ist es, ein verbessertes medizinisches Instrument zur atraumatischen Behandlung von Hohlorganen vorzusehen.

Diese Aufgaben werden mit einer Vorrichtung gemäß dem Patentanspruch 1 gelöst. Vorteilhafte Ausgestaltungen hiervon sind in den Unteransprüchen angegeben.

Erfindungsgemäß ist eine rohrförmige Hülse zur atraumatischen Behandlung von Hohlorganen vorgesehen, wobei die Hülse in einem Ausgangszustand gefaltet ist und in einem Endzustand zum Anliegen an einer Innenwandung eines Hohlorganes entfaltbar ist.

Die Hülse zeichnet sich dadurch aus, dass die Hülse aus einer Außenwandung und einer Innenwandung ausgebildet ist, die konzentrisch zueinander angeordnet sind, wobei die Außenwandung und die Innenwandung stirnseitig derart dicht miteinander verbunden sind, dass im Bereich zwischen der Außenwandung und der Innenwandung ein in etwa röhrenförmiges Kompartiment ausgebildet ist und die Faltung der Hülse um eine Hülsenlängsachse gerichtet ist.

Im Kompartiment können ein oder mehrere Füllstoffe für verschiedene medizinische Anwendungen und/oder zur Verbesserung mechanischer Eigenschaften anordbar sein. Verschiedene Arten von Füllstoffen, die zur Anordnung im Kompartiment geeignet sind, werden nachfolgend noch detailliert beschrieben,

Dadurch, dass die Faltung der Hülse um eine Hülsenlängsachse gerichtet ist, ist es möglich, eine rohrförmige Hülse bereitzustellen, bei der der Ausgangsdurchmesser in gefaltetem Zustand äußerst gering ist. Dies ermöglicht ein relativ einfaches und sicheres Einbringen der Hülse im gefalteten Zustand zum Implantationsort aufgrund des geringen Außendurchmessers. Insbesondere ist vorgesehen, dass sowohl die Innenwandung als auch die Außenwandung im selben Faltmuster gefaltet sind. Weiterhin ist der Ausgangsdurchmesser der gefalteten rohrförmigen Hülse wesentlich kleiner als der am Implantationsort geforderte Außendurchmesser nach der Entfaltung der Hülse.

Das Kompartiment der rohrförmigen Hülse kann im Rahmen der vorliegenden Erfindung ein einziges Kompartiment oder auch mehrere Kompartimente oder auch mehrere (bis multiple) Mikrokompartimente umfassen.

Im Rahmen der vorliegenden Erfindung wird unter einem in etwa rohrförmigen Kompartiment ein Raum verstanden, der durch die Innen- und Außenwandung begrenzt ist. Die Form dieses Raums ist nicht auf eine zylindrische Ringform begrenzt, sondern die Dicke des Kompartiments kann sowohl in Längsrichtung der rohrförmigen Hülse als auch quer zur Längsrichtung variieren und von Wandungen, Stegen oder dergleichen unterbrochen sein.

Die rohrförmige Hülse kann eine gitterartige Mantelwandung aufweisen. Das bedeutet, in der Mantelwandung können Fehlstellen ausgebildet sein. Dennoch weisen die einzelnen Gitterstreben einer derartigen gitterförmigen Mantelwandung ebenfalls zumindest eines oder auch mehrere Kompartimente auf.

Die rohrförmige Hülse kann eine geschlossene Mantelwandung aufweisen. Das bedeutet die Hülse weist eine durchgehende geschlossenes Außenfläche auf in der keine Fehlstellen vorhanden sind.

Die Außenwandung und/oder die Innenwandung bzw. eine innere und eine äußere Mantelwandung der Hülse können im Wesentlichen geschlossen ausgebildet sein,

Unter einer geschlossen ausgebildeten Mantelwandung wird im Rahmen der vorliegenden Erfindung eine Wandung verstanden, die vollständig geschlossen bzw. porenfrei sein oder eine geringe Porosität aufweisen kann, wobei die in der Hülse ausgebildeten Poren maximal einen Durchmesser kleiner 100 µm, bzw. kleiner 80 µm, bzw. kleiner 60 µm, bzw. kleiner 40 µm, bzw. kleiner 30 µm bzw. kleiner 10 µm und vorzugsweise kleiner 20 µm aufweisen. Die angegebenen Werte können sich auch auf eine maximale Porenweite einer elliptischen oder andersförmig ausgebildeten Pore beziehen, wobei die Werte dann die breitesten Weite der Porenöffnung betreffen.

Bei einer derartigen Porengröße wird verhindert, dass Blutkörperchen, wie z.B. Blutplättchen (Thrombozyten), in die Hülse eintreten, was einige der vorstehend beschriebenen Nachteile, insbesondere die Thrombogenität, d. h. die Neigung zur Blutgerinnselbildung, im Bereich der behandelten Gefäßläsion vermindert. Durch die vollständige Abdeckung der Läsion durch die Hülse wird die Interaktion mit Blut und Blutkörperchen vermieden, die sonst fortlaufend stattfinden würde. Darunter ist insbesondere die Anlagerung und Aktivierung von Blutkörperchen zu verstehen. Insbesondere wird die Aktivierung von Blutplättchen und damit die Aktivierung des Gerinnungssystems reduziert.

Bei den angegebenen Werten kann es zwar vorkommen, dass ein paar wenige Blutkörperchen in die Hülse eintreten und/oder in den Poren stecken bleiben. Trotzdem wird die Aktivierung von Blutplättchen und des Gerinnungssystems deutlich reduziert, weil keine fortlaufende Interaktion mit neuen Blutplättchen stattfindet.

Eine oder beide Wandungen der gitterartigen Mantelwandungen können ebenfalls eine im Wesentlichen geschlossen ausgebildete Mantelwandung und demgemäß Poren aufweisen.

Die erfindungsgemäße Hülse ist auf einem Ballonkatheter anordbar. Die Hülse wird dann zusammen mit dem Ballonkatheter zum Implantationsort bewegt. Durch Expansion des Ballonkatheters wird die Hülse entfaltet und bildet eine geschlossene tubuläre Abdeckung des aufgedehnten, vormals verengten oder verschlossenen Hohlraums aus.

Dadurch, dass die Hülse in gefaltetem Zustand auf einem Ballonkatheter anordbar ist, weist sie beim Eindringen in den menschlichen Körper einen geringen Außendurchmesser auf. Durch das Entfalten am Implantationsort wird der Außendurchmesser bzw. der Umfang entsprechend vergrößert. Durch die Faltung ist hierfür jedoch keine größere plastische Dehnung des Hülsenmaterials erforderlich. Somit kommt es bei der erfindungsgemäßen Hülse im Gegensatz zu ballonexpandierbaren Stents auch nicht zur Ausbildung von Rückstellkräften, die zu einem Zurückfedern auf einen geringeren Durchmesser führen (Recoil). Eine derartige sich wieder zurückstellende Aufweitung von Stents führt zu einer ersten Wiederverengung der behandelten Läsion.

Wie bereits vorstehend beschrieben, ist erfindungsgemäß vorgesehen, dass der Außendurchmesser der rohrförmigen Hülse nach dem Entfalten in etwa dem Durchmesser einer Innenwandung eines Hohlorganes entspricht.

Einrisse in der Wandung des Hohlorganes, die durch die Expansion bzw. die Inflation des Ballonkatheters entstanden sind, werden durch die entfaltete, die Läsion komplett abdeckende Hülse von direktem Kontakt mit Blut ausgeschlossen. Auf diese Weise wird die Thrombogenität, d. h. die Neigung der Blutgerinnselbildung, im Bereich der behandelten Gefäßläsion vermindert.

Vorzugsweise kann die Faltung der rohrförmige Hülse als Plissierung ausgebildet bzw. die Hülse plissiert sein. Unter Plissieren wird im Rahmen der vorliegenden Erfindung ein Faltenlegen und Pressen der rohrförmigen Hülse verstanden. Durch das Plissieren ist es möglich, den Außendurchmesser der rohrförmigen Hülse im nicht-entfalteten Zustand zu reduzieren. Optional kann ein Adhäsiv auf die gefaltete Hülse aufgebracht sein, um die Faltung zu stabilisieren und damit den geringen Durchmesser beim Transport zum Implantationsort beizubehalten.

Die Faltung der Hülse ist um eine Hülsenlängsachse gerichtet, wobei die Faltung sowohl in als auch entgegen dem Uhrzeigersinn erfolgen kann. Das Falten und Plissieren erfolgt ähnlich dem in der WO 02 076 700 A1 anhand eines Ballonkatheters beschriebenem Prinzip.

Die rohrförmige Hülse kann auch mit einem medizinischen Wirkstoff, wie z. B. einer Hydrogelschicht, dotiert sein. Die Hydrogelschicht weist bevorzugt eine Dicke von ca. 5 µm bis 20 µm, bzw. von 7,5 bis 12,5 µm und insbesondere von 10 µm auf. Die Hydrogelschicht ist z. B. mit einem Antiproliferativum, z. B. Paclitaxel oder Everolimus, beladen.

Durch das Dotieren der Außenfläche der Hülse mit einer Hydrogelschicht klebt die Hülse aufgrund der Hydrogelschicht nach der Entfaltung an einer Innenwandung eines Hohlorganes, insbesondere eines Blutgefäßes, an.

Zudem kann ein biokompatibler Kleber, wie z.B. ein Hydrogel, den Faltungszustand der Hülse während des Transports zum Implantationsort stabilisieren.

Die Hülse kann aus einer Folie oder einer textilen Struktur ausgebildet sein. Als Materialien eignen sich bspw. biokompatible Kunststoffe, insbesondere bioabbaubare Polymere wie die Polylactide.

Die textile Struktur kann aus Fäden gewoben, gewirkt, geflochten, gestrickt und/oder geklöppelt sein oder aus einem Vlies ausgebildet sein.

Ein Vlies ist ein Gebilde aus Fasern begrenzter Länge, Endlosfasern (Filamenten) oder geschnittenen Garnen jeglicher Art und jeglichen Ursprungs, die auf irgendeine Weise zu einem Vlies (einer Faserschicht, einem Faserflor) zusammengefügt und auf irgendeine Weise miteinander verbunden worden sind.

Vliesstoffe sind größtenteils flexible textile Flächengebilde, d. h. sie sind leicht biegsam, ihre Hauptstrukturelemente sind textile Fasern und sie weisen eine vergleichsweise geringe Dicke gegenüber ihrer Länge und Breite auf. Ebenso existieren Vliesstoffe, die wegen der verwendeten Fasern oder der Verfestigungsverfahren eher Papieren, Folien oder faserverstärkten Kunststoffen als Textilien ähneln. Vliesstoffe stellen eine Materialgruppe mit einer großen Eigenschaftsvielfalt dar, die einem breiten Spektrum von medizinischen Anwendungsanforderungen gezielt angepasst werden kann.

Die textile Struktur kann auch durch Verkreuzen bzw. Verschlingen von Garnen, wie es beim Weben, Wirken, Stricken, der Spitzenherstellung, dem Flechten und Herstellung von getufteten Erzeugnissen geschieht, ausgebildet sein.

Bei einer Ausgestaltung der Hülsenwandung aus einem Vlies werden multiple polymorphe MikroKompartimente ausgebildet, die eine kapillare Sog- bzw-Saugwirkung aufweisen, und damit passiv einen flüssigen Binder (oder Blutplasma) ansaugen können - und auf diese Weise eine in-situ-Vernetzung bewirken.

Weiterhin kann die Hülse zylindrisch ausgebildet sein. Alternativ kann die Hülse auch jede aus dem Stentbereich bekannte Form aufweisen. D.h. die Hülse kann konisch, verzweigt, eingeschnürt (nach Art einer Eieruhr), elliptisch oder kreisförmig in einer Seitenansicht ausgebildet sein.

Das Material, aus dem die Hülse ausgebildet ist, und ihre Beschichtung weisen vorzugsweise eine gewisse Anschmiegsamkeit bzw. Anlegbarkeit auf, um sich an die Struktur einer Wandung bzw. die Oberflächenbeschaffenheit eines Hohlorganes anzupassen und auf dies Weise Läsionen abzudecken.

Zudem besitzt das Material vorzugsweise eine geringe Elastizität, d. h. es ist elastisch nur innerhalb gewisser Grenzen verformbar, um bei einer nachfolgend beschriebenen Ausführungsform der rohrförmigen Hülse mit einer gewissen Festigkeit einen Recoil zu verhindern.

In dem Kompartiment können als Füllstoff Nanopartikel oder Textilfasern oder ein Vlies angeordnet sein. Weiterhin kann im Kompartiment als Füllstoff eine klebrige, pastöse Masse angeordnet sein.

Demgemäß können sowohl die Außenwandung als auch die Innenwandung und der Füllstoff aus einem Vlies, vorzugsweise aus ein und demselben Vlies oder aber auch aus zwei verschiedenen Vliesstoffen ausgebildet sein. Eine Außenfläche des Vlieses bildet dann die Außenwandung und eine Innenfläche des Vlieses bildet dann die Innenwandung der rohrförmigen Hülse aus.

Die Zwischenräume zwischen Textilfasern des Vliesstoffes stellen dann Mikrokompartimente im Sinne der vorliegenden Erfindung dar. In diesen Mikrokompartimenten ist ebenfalls ein Füllstoff, wie z.B. ein Bindemittel anordbar oder es ist möglich, dass Blutplasma in die Mikrokompartimente eintritt und als Bindemittel wirkt.

Die Dicke der Außenwandung und der Innenwandung kann jeweils ca. 20 µm bis 30 µm betragen. Die Dicke des Kompartiments quer zur Längsrichtung bzw. in radialer Richtung kann ebenfalls ca. 20 µm bis 30 µm betragen.

Weiterhin kann das geschlossene röhrenförmige Kompartiment in zwei oder mehrere oder multiple Unter-Kompartimente unterteilt sein, die mit unterschiedlichen Komponenten gefüllt sind, bspw. mit einem Präpolymer oder Polymer (Textilfasern, Partikel, Pulver oder Vlies), und mit einem Vernetzungsmittel oder Klebemittel.

Hierbei ist vorgesehen, dass die räumliche Trennung der verschiedenen Komponenten durch die Unterkompartimente durch Expansion des Ballonkatheters aufgehoben wird, indem Trennstrukturen zerstört werden, so dass die unterschiedlichen Komponenten miteinander in Kontakt treten. Auf diese Weise erfolgt dann eine mechanische Verfestigungs- oder eine chemische Vernetzungsreaktion, die der entfalteten Hülse eine mechanische Festigkeit verleiht.

Zwischen der Innenwandung und der Außenwandung kann eine Zwischenwandung derart vorgesehen sein, dass zwei koaxial verlaufende Kompartimente ausgebildet sind. Grundsätzlich sind auch zwei oder mehr Zwischenwandungen möglich, so dass mehrere koaxial verlaufende Kompartimente ausgebildet werden.

Bei einer solchen Ausführungsform soll, wobei die Zwischenwandung einen geringeren Entfaltungsdurchmesser aufweist als die innere und die äußere Wandung, durch den Ballondruck die Zwischenwandung kontrolliert reißen. Auf diese Weise wird dann eine Verbindung zwischen beiden Kompartimenten hergestellt.

Zwischen der Innenwandung und der Außenwandung kann eine Zwischenwandung derart vorgesehen sein, dass zwei in Längsrichtung hintereinander angeordnete Kompartimente ausgebildet sind. Grundsätzlich sind auch zwei oder mehr Zwischenwandungen möglich, so dass mehrere in Längsrichtung hintereinander angeordnete Kompartimente ausgebildet werden.

Die Wandungen der Hülse können durch Verbindungspunkte oder -linien miteinander verbunden sein, die bspw. durch Kleben oder Verschweißen hergestellt sind.

Derartige Verbindungspunkte oder -linien bewirken einen besseren Zusammenhalt zwischen den Wandungen während des Plissier-, Faltungs- und Entfaltungsvorganges. Die Verbindungspunkte oder -linien können als ein auf der Zylindermantelfläche angeordnetes Muster von Verbindungspunkten, welches z. B. durch Laserschweißung erzeugt wurde, sein.

Die Hülse bzw. zumindest eines der Kompartimente kann einen Zuführkanal aufweisen, über den eine flüssige Komponente von außen dem Kompartiment zuführbar ist.

Im Entfaltungszustand der Hülse kann eine in situ-Polymerisation mindestens zweier Komponenten in einem oder mehreren der abgeschlossenen röhrenförmigen Kompartimente stattfinden.

Eine solche in situ-Polymerisation führt zu einer mechanischen Stabilisierung der entfalteten Hülse, was ein Rekollabieren in den gefalteten Zustand verhindert. Die Polymerisation sollte bei einem Ballondruck von bis zu 25 atm möglich sein und Reaktionszeiten von 10 bis 15 Sekunden nicht übersteigen.

Die Polymerisation kann bewirkt werden durch
- eine mechanische Zusammenführung der Komponenten (z.B. PLA-Pulver oder PLA-Mikrosphären 1-2 µm und Binder bzw. Vernetzer) durch Penetration von Trennwandungen zwischen den Kompartimenten, oder
- eine fluidmechanische aktive Injektion oder ein passives Aufsaugen eines flüssigen Vernetzungsmittels, oder
- ein mechanisches und thermisches Einwirken von Ultraschall, oder
- eine optisch-thermische Beaufschlagung, oder
- eine thermische Beaufschlagung, oder
- eine chemische Reaktion, z.B. durch sog. Klick-Chemie, wie der Thiol-En-Reaktion.

Für ein mechanisches und thermisches Einwirken mittels Ultraschall kann bspw. eine UltraschallSonde in den Ballonkatheter integriert oder diesem zuführbar sein.

Eine optisch-thermische Beaufschlagung kann bspw. mittels eines Lasers erfolgen, der in den Ballonkatheter integriert oder diesem zuführbar ist. Durch das Einwirken von UV-Licht (ca. 275 nm) auf die entsprechende Komponente kann eine Lasersinterung von PLA-Pulver erfolgen.

Eine thermische Beaufschlagung kann z.B. mittels einer Hochfrequenz-Sonde, die in den Ballonkatheter integriert oder diesem zuführbar ist, erfolgen.

Eine chemische Reaktion, z.B. durch sog. Klick-Chemie, wie der Thiol-En-Reaktion, kann z.B. durch Photopolymerisation und Integration eines UV-Lasers in den Ballonkatheter erfolgen.

Die Wandungen der Hülse können derart miteinander verbunden sein, bspw. mittels Laserschweißen, dass Kanäle ausgebildet sind, die ein Muster, z.B. ein Rautenmuster, aufweisen. Dieses Muster kann so angelegt sein, dass strebenförmige Kanäle entstehen, die ähnlich den Streben eines Stents verlaufen.

Die Kanäle bzw. die Kompartimente bzw. die Unter-Kompartimente bzw. die Zwischenräume können mit einer vernetzbaren Polymerkomponente oder einer mechanisch unter Ballondruck verfestigbaren Komponente gefüllt sein.

Die Kanäle bzw. die Kompartimente bzw. die Unter-Kompartimente können mit einem Füllstoff, insbesondere einer Trockensubstanz, wie z. B. einem Polymer-Nanopulver gefüllt sein, wobei Zwischenräume zwischen dem Pulver mit einem flüssigen Vernetzungsmittel oder einem flüssigen Bindemittel gefüllt sind.

Hierbei ist vorgesehen, dass die Komponenten miteinander in Kontakt treten, sobald die räumliche Trennung der Komponenten durch Expansion des Ballonkatheters aufgehoben wird, da die entsprechenden Trennwandungen zerstört werden.

Die Außenfläche der Hülse kann mit einem medizinischen Wirkstoff, wie z.B. einem Antiproliferativum, und bzw. oder einer einheilungsfördernden Substanz, wie z.B. Fibringel, dotiert sein, wobei dieses optional mit autologen Zellen eines Patienten vermischt sein kann.

Weiterhin kann eine Dotierung der Innenfläche der Hülse mit einer antithrombogenen Substanz, wie z.B. Heparin, und bzw. oder einem Wirkstoff erfolgen, der eine Endothelzellen-Besiedlung fördert.

Zudem kann eine Vorbehandlung bzw. ein Priming der Innen- und Außen-Oberfläche der Hülse unmittelbar vor der Implantation erfolgen, indem die Oberfläche mit körpereigenen Substanzen des Empfängerpatienten (z.B. Vollblut, Plasma, Serum, Zellen) benetzt wird.

Eine Benetzung der Innen- und/oder Außen-Oberfläche/n der Hülse während des Implantationsvorganges ("in situ") mit bioaktiven Wirkstoffen oder körpereigenen Substanzen des Empfängerpatienten ist ebenfalls möglich, indem diese über entsprechende Zuführkanäle über eines oder mehrere Kompartimente injiziert werden, die in Öffnungen der Innen- und/oder Außenflächen der Hülse münden.

Die Haftung der Substanzen kann durch Oberflächenstrukturierung oder Gelfilm-Auflagerung der Oberflächen der Hülse verbessert werden.

Insbesondere ist erfindungsgemäß ein System zur atraumatischen Behandlung von Hohlorganen vorgesehen, das einen Ballonkatheter und eine rohrförmige Hülse umfasst.

Zudem kann bei diesem System auf der Hülse im Ausgangszustand eine äußere Schutzhülle angeordnet sein.

Durch eine solche folienartige Schutzhülle wird ein Blutkontakt vermieden und somit eine Thrombusbildung während des Einbringens der Hülse verhindert. Am Implantationsort kann die Schutzhülle dann durch Zurückziehen oder durch ein entfaltungsbedingtes Zerreißen entfernt werden. Darüber hinaus kann die Stabilität der Faltung der Hülse für den Transport auf dem Ballonkatheter durch eine adhäsive Oberflächenbehandlung der Außenfläche der Hülse unterstützt werden. Die erfindungsgemäße Hülse umfasst einen strukturierten Wandaufbau mit Kompartimentierung. Das bedeutet, es ist zumindest ein Kompartiment oder es sind mehrere durch Wandungen oder Stege voneinander getrennte Kompartimente oder auch Mikrokompartimente vorgesehen. Ein Kompartiment im Sinne der vorliegenden Erfindung ist somit ein umschlossener Raum, der mit einem Füllstoff wie z.B. einer Flüssigkeit, einem Pulver und/oder einem Gel usw. gefüllt werden kann.

Die Hülse kann weiterhin eine geschlossene oder eine poröse Mantelfläche aufweisen, wobei Verbindungspunkte- und -linien zwischen Innen- und Außenhülle vorgesehen sind. Die Hülse kann auch als eine Gitterstruktur mit einer Mantelwandung mit oder ohne Poren ausgebildet sein.

Im Folgenden wird die Erfindung anhand der in den Figuren dargestellten Ausführungsbeispiele näher beschrieben. Diese zeigen in:
- Figur 1: eine plissierte gefaltete Hülse in einer Draufsicht von vorne gemäß einer ersten Ausführungsform der erfindungsgemäßen Vorrichtung,
- Figur 2: eine perspektivische Ansicht von der Seite der erfindungsgemäßen Hülse im entfalteten Zustand,
- Figur 3: eine perspektivische seitliche Ansicht der erfindungsgemäßen Hülse auf einen Ballonkatheter mit inflatiertem Ballon,
- Figur 4: eine weitere Ausführungsform einer erfindungsgemäßen Hülse in einer seitlich geschnittenen Ansicht,
- Figur 5: eine weitere Ausführungsform einer erfindungsgemäßen Hülse in einer seitlich geschnittenen Ansicht,
- Figur 6: eine Zwischenwandung der in Figur 5 dargestellten Hülse in einer perspektivischen Ansicht,
- Figur 7: eine weitere Ausführungsform der erfindungsgemäßen Hülse in einer seitlichen perspektivischen Ansicht, auf einen Ballonkatheter mit inflatiertem Ballon,
- Figur 8: eine Detailansicht der Mantelwandung der in Figur 7 dargestellten Ausführungsform der erfindungsgemäßen Hülse,
- Figur 9: eine perspektivische Detailansicht der Mantelwandung der in Figur 2 dargestellten Hülse,
- Figur 10: eine perspektivische Detailansicht der Mantelwandung eines weiteren Ausführungsbeispiels einer Mantelwandung, bei der Innen- und Außenwandung über Verbindungspunkte miteinander verbunden sind,
- Figur 11: eine perspektivische Detailansicht der Mantelwandung eines weiteren Ausführungsbeispiels einer Mantelwandung, bei der Innen- und Außenwandung über Verbindungspunkte miteinander verbunden sind, wobei zumindest in der Außenwandung Öffnungen bzw. Poren vorgesehen sind,
- Figur 12: eine perspektivische Detailansicht der Mantelwandung eines weiteren Ausführungsbeispiels einer gitterartigen Mantelwandung,
- Figur 13: eine perspektivische Detailansicht der Mantelwandung eines weiteren Ausführungsbeispiels einer gitterartigen Mantelwandung, wobei zumindest in der Außenwandung Öffnungen bzw. Poren vorgesehen sind,
- Figur 14: eine perspektivische Detailansicht der Mantelwandung eines weiteren Ausführungsbeispiels einer Mantelwandung, wobei an der Mantelwandung außenseitig sich in Richtung eines Hohlorganes erstreckende Zapfen zur Bindung eines medizinischen Wirkstoffs vorgesehen sind, und
- Figur 15: eine perspektivische Detailansicht der Mantelwandung eines weiteren Ausführungsbeispiels einer gitterartigen Mantelwandung,

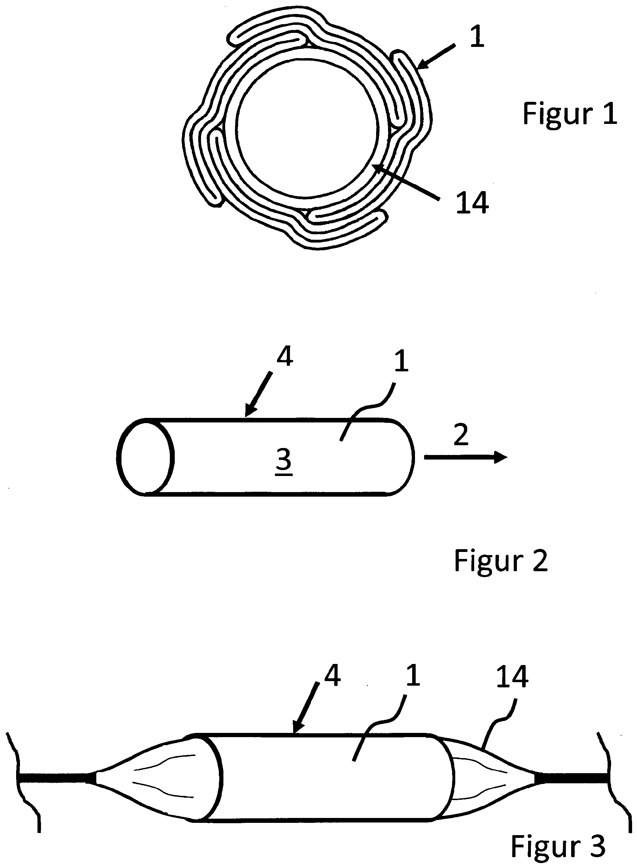

Eine rohrförmige Hülse 1 zur atraumatischen Behandlung von Hohlorganen ist gemäß einem ersten Ausführungsbeispiel zylindrisch aus einer einzigen Wandung ausgebildet (Figur 1 bis Figur 3).

Die rohrförmige Hülse 1 bzw. deren Wandung 3 ist in einem Ausgangszustand plissiert und gefaltet. Die Faltung der Hülse ist um eine Hülsenlängsachse 2 gerichtet. Weiterhin erfolgt die Faltung im oder gegen den Uhrzeigersinn um die Hülsenlängsachse 2.

Die Wandung ist aus einer Folie, wie z. B. Polylactid (PLA), ausgebildet. Die Folie weist eine Dicke von ca. 20 µm bis 90 µm, bzw. von ca. 40 µm bis 70 µm und insbesondere von 50 µm auf.

Weiterhin ist die äußere Wandung bzw. die Mantelwandung 3 mit einem hoch-pastösen Hydrogel derart beschichtet, dass auf der Mantelwandung 3 vollflächig eine Hydrogelschicht 4 ausgebildet ist. Die Hydrogelschicht 4 weist bevorzugt eine Dicke von ca. 10 µm bis 50 µm auf und ist z.B. mit einem Antiproliferativum, wie z.B. Paclitaxel oder Everolimus, beladen bzw. dotiert. Ein bevorzugter Dotierungsbereich beträgt für Everolimus 7 µg/mm bis 10 µg/mm in einer 1:1-Mischung mit der Hydrogelmatrix.

Bei dieser Ausführung der Hülse, bei der die Mantelwandung nur aus einer einlagigen Folie ausgebildet ist, wird eine dickere Hydrogelschicht mit einer Dicke von z.B. 40 µm, vorgesehen, um Unregelmäßigkeiten einer Gefäßinnenwandung auszugleichen und um die Kontaktmantelfläche zu vergrößern. Bei mehrlagigen Wandungen kann die Dicke ca. 10 µm bis 30 µm bzw. ca. 15 µm bis 20 µm betragen.

Die rohrförmige Hülse wird in einem plissierten und gefalteten Ausgangszustand auf einem zusammengefalteten Ballonkatheter 14 angeordnet. Die Hülse kann nach ihrer Faltung auf einem entfernbaren Kern durch Überstreifen auf dem gefalteten Ballon eines Ballonkatheters angeordnet werden. Alternativ kann eine Faltung direkt auf dem Ballonkatheter erfolgen, wobei sich der Ballon des Ballonkatheters bereits in einem gefalteten Zustand befindet. Alternativ ist es auch möglich, Ballonfolie und Hülse gemeinsam in einem Arbeitsschritt zu plissieren und zu falten. Dieses Vorgehen fördert den Zusammenhalt zwischen Ballonfolie und Hülse während des Transports zum Implantationsort der Hülse. In allen Fällen kann die Faltung der Plissuren der Hülse unter Zuhilfenahme eines Adhäsivs erfolgen, um diese für die Phase des Transportes der Hülse zum Implantationsort zu stabilisieren. Jedoch sollte nur eine geringe Adhäsiv-Haftung zwischen Ballonkatheter und Hülse bereitgestellt werden, damit sich die Hülse nach dem auf das Inflatieren des Ballonkatheters folgenden Deflatierungsvorgangs leicht von der Oberfläche des Ballons löst. Der Ausgangsdurchmesser der gefalteten rohrförmigen Hülse ist wesentlich kleiner als der am Implantationsort geforderte Außendurchmesser nach der Entfaltung der Hülse 1.

Die Stabilität der Faltung und der Plissur der Hülse 1 beim Transport bzw. beim Einbringen in das Hohlorgan kann durch eine adhäsive Oberflächenbehandlung der Außenwandung der Hülse unterstützt werden.

Am Implantationsort wird die rohrförmige Hülse 1 durch die Expansion des Ballonkatheters 14 in einem Endzustand derart entfaltet, dass die Hülse 1 entfaltet und entplissiert ist.

Dabei wird die zylindrische Mantelwandung 3 von innen gegen das betroffene Wandsegment einer Innenwandung eines Hohlorganes gepresst. Aufgrund der Klebe-Eigenschaft der Hydrogelschicht 4 haftet die rohrförmige Hülse 1 selbstständig an der Innenwandung des Hohlorganes, insbesondere eines Blutgefäßes, an. Durch die Faltung ist hierfür jedoch keine größere plastische Dehnung des Hülsenmaterials erforderlich. Somit kommt es bei der erfindungsgemäßen Hülse 1 im Gegensatz zu ballonexpandierbaren Stents auch nicht zu einem Recoil. D.h. die Hülse wird durch die Inflation des Ballons lediglich auf einen Enddurchmesser entfaltet, der im Wesentlichen dem Innendurchmesser der betroffenen Gefäßwandung entspricht. Die Hülse 1 legt sich dann als dünnwandiges röhrenförmiges Implantat an die Gefäßwandung an.

Mittels der die Läsion komplett abdeckenden Hülse werden Gefäßwandeinrisse im Blutgefäß, die durch die Ballonexpandierung entstanden sind, vom direkten Kontakt mit Blut ausgeschlossen.

Auf diese Weise wird die Thrombogenität bzw. die Neigung zur Blutgerinnselbildung im Bereich der behandelten Gefäßläsion herabgesetzt.

Insbesondere ist vorgesehen, dass der im Hydrogel eingelagerte Wirkstoff je nach Dosierung und Art der Einbettung in das Hydrogel über einen längeren Zeitraum von ca. drei bis fünf Monaten auf die Gefäßwand derart einwirkt, dass die reaktive Proliferation von Gefäßwandzellen (als Reaktion auf das Trauma) reduziert wird.

Sofern die Hülse aus Polylactid ist, wird sie nach einem bestimmten Zeitraum, bevorzugt nach drei bis fünf Monaten, abgebaut.

Bei der Deflation des Ballonkatheters löst sich die Hülse von der Ballonaußenfläche ab und haftet dann an einer Innenwandung des Hohlorganes, z. B. der Blutgefäßwandung, als dünnwandiges rohrförmiges Implantat an.

Alternativ kann die Hülse aus einer dünnwandigen, textilen Struktur ausgebildet sein. Eine derartige Struktur kann aus Fäden gewoben, gewirkt, geflochten, gestrickt oder geklöppelt sein. Zudem ist auch eine Ausführungsform aus einem röhrenförmigen Vlies (nonwoven) möglich.

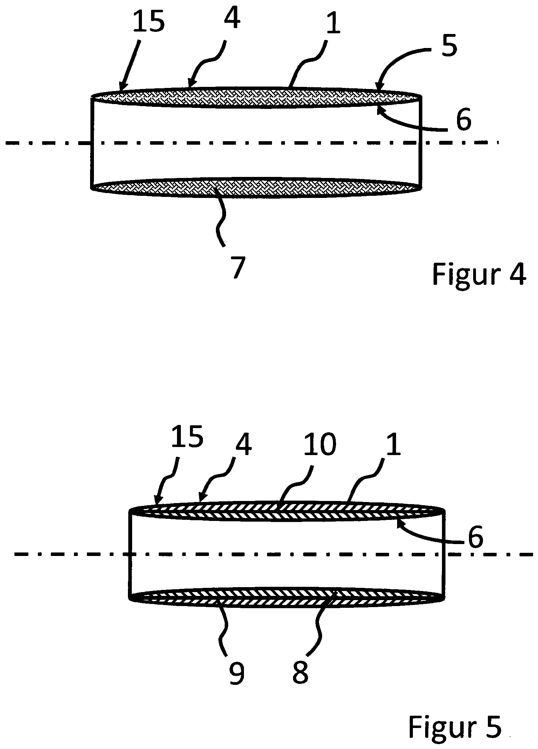

Gemäß einer Ausführungsform der vorliegenden Erfindung ist die rohrförmige Hülse 1 aus zwei konzentrisch angeordneten zylindrischen Wandungen ausgebildet, wobei die äußere Wandung eine Außenwandung 5 ausbildet und die innere Wandung eine Innenwandung 6 ausbildet (Figur 4).

Sofern nichts anderes beschrieben ist, entspricht diese Ausführungsform der vorstehend beschriebenen Ausführungsform.

Hierbei ist vorgesehen, dass Endbereiche bzw. in Längsrichtung 2 der Hülse 1 vorne und hinten liegende Stirnseiten der Außenwandung 5 und der Innenwandung 6 dicht miteinander, bspw. mittels Schweißen, verbunden sind. Auch eine einstückige Verbindung der Außenwandung 5 und der Innenwandung 6 ist möglich.

Auf diese Weise wird im Bereich zwischen der Außenwandung 5 und der Innenwandung 6 ein geschlossenes, im Wesentlichen röhrenförmiges Kompartiment 7 ausgebildet (Figur 4). Die Außenwandung 5 und die Innenwandung 6 sind ebenfalls, wie bereits vorstehend beschrieben, vorzugsweise aus einer Folie, aus z.B. Polylactid (PLA), ausgebildet.

Die Dicke der Außenwandung 5 und der Innenwandung 6 beträgt jeweils ca. 20 µm bis 30 µm. Die Dicke des Kompartimentes 7 quer zur Längsrichtung bzw. in radialer Richtung 2 beträgt ebenfalls ca. 20 µm bis 30 µm.

Im Kompartiment 7 ist eine dünne Schicht aus einer biokompatiblen Substanz angeordnet. Die Substanz kann ein Gemisch aus Polylactid-Nano-Mikrofasern mit einem Faserdurchmesser von 100 nm und einer Faserlänge von 2 µm bis 5 µm und einem Klebe- bzw. Bindemittel, wie z. B. einem hochviskosen Hydrogel, sein.

Während der durch den Ballonkatheter 14 bewirkten Entfaltung der rohrförmigen Hülse 1 wirkt aufgrund des Anliegens der Außenwandung 5 an einer Innenwandung eines Hohlorganes und des vom Ballonkatheter auf die Innenwandung 6 übertragenen Drucks ein Kompressionsdruck auf die im Kompartiment 7 angeordnete biokompatible Substanz. Der Kompressionsdruck kann dabei über 20 atm betragen, wodurch sich die Mikrofasern in etwa in axialer Richtung, d.h. in Richtung der Hülsenlängsachse 2, ausrichten und sich in Verbindung mit dem Kleber verfestigen. Auf diese Weise wird im entfalteten und entplissierten Endzustand der rohrförmigen Hülse 1 eine steife Struktur ausgebildet. Eine derartige steife Struktur bewirkt, ähnlich wie die eingangs beschriebenen Stents, eine Schienung und Offenhaltung des Hohlorganes.

Alternativ kann das Kompartiment 7 nur mit Polylactid-Nano-Mikrofasern gefüllt sein. Die innere und/oder die äußere Wandung 5, 6 der Hülse können dann eine Mikroperforation aufweisen, d. h., dass die Außenwandung 5 und die Innenwandung 6 eine Porosität aufweisen, wobei die in der Innenwandung 6 und in der Außenwandung 5 ausgebildeten Poren maximal einen Durchmesser kleiner 100 µm, bzw. kleiner 80 µm, bzw. kleiner 60 µm, bzw. kleiner 40 µm, bzw. kleiner 30 µm bzw. kleiner 10 µm und vorzugsweise kleiner 20 µm aufweisen.

Auf diese Weise kann während des Entfaltens und Entplissierens der rohrförmigen Hülse 1 Körperflüssigkeit, insbesondere Blutplasma, in das Kompartiment 7 eintreten. Der Eintritt von Körperflüssigkeit in das Kompartiment 7 kann dadurch verstärkt werden, dass der Ballonkatheter mehrmals inflatiert und deflatiert wird, so dass die Außenwandung 5 und die Innenwandung 6 der Hülse 1 mehrfach in direkten Blutkontakt treten. Das einströmende Plasma kann aufgrund seines Protein- und Fibrinogengehaltes eine ähnliche Wirkung wie ein synthetischer Kleber aufweisen. Auf diese Weise erfährt die rohrförmige Hülse 1 in ihrem entfalteten Zustand ebenfalls eine mechanische Stabilisierung.

Gemäß einer bevorzugten Ausführungsform ist die Hülse aus einer Vliesstruktur ausgebildet, wobei zwischen den Einzelnen fasern Mikrokompartimente ausgebildet sind. Aus der Mikroporosität und Mikrokompartimentierung der fasern bzw. einer solchen Hülse resultiert ein passiver Ansaugeffekt von Blutplasma nach einer Implantation im Blutgefäß.Weiterhin ist das Vlies mit Calciumcloridsalz dotiert, wodurch eine Fibrinpolymerisation induziert wird.

Gemäß einer Ausführungsform der vorliegenden Erfindung weist die rohrförmige Hülse 1 zumindest zwei Kompartimente, insbesondere ein inneres Kompartiment 8 und ein äußeres Kompartiment 9 auf (Figuren 5, 6 und 9).

Sofern nichts anderes beschrieben ist, entspricht diese Ausführungsform im Wesentlichen der zweiten Ausführungsform der vorliegenden Erfindung.

Eine solche rohrförmige Hülse 1 weist zwischen der Außenwandung 5 und der Innenwandung 6 eine Zwischenwandung 10 auf, die sich ebenfalls in Hülsenlängsrichtung 2 erstreckt. Die Außenwandung 5, die Zwischenwandung 10 und die Innenwandung 6 sind jeweils aus einer Folie aus einem der vorstehend beschriebenen Materialien ausgebildet. Die Endbereiche bzw. Stirnseiten der Folien sind dicht miteinander, z. B. mittels Schweißen, verbunden.

Demgemäß bildet eine rohrförmige Hülse 1 gemäß dieser Ausführungsform das äußere Kompartiment 9 zwischen der Außenwandung 5 und der Zwischenwandung 10 und das innere Kompartiment 8 zwischen der Zwischenwandung 10 und der Innenwandung 6 aus.

Das innere Kompartiment 8 und das äußere Kompartiment 9 verlaufen koaxial zueinander bezüglich der Hülsenlängsachse 2. Im äußeren Kompartiment 9 sind PLA-Fasern oder ein PLA-Pulver angeordnet. Im inneren Kompartiment 8 liegt ein Vernetzungsmittel in flüssiger Form vor.

Hierbei kann vorgesehen sein, dass die Zwischenwandung 10 eine geringere Dicke und einen geringeren Entfaltungsdurchmesser als die Außenwandung 5 und die Innenwandung 6 aufweist.

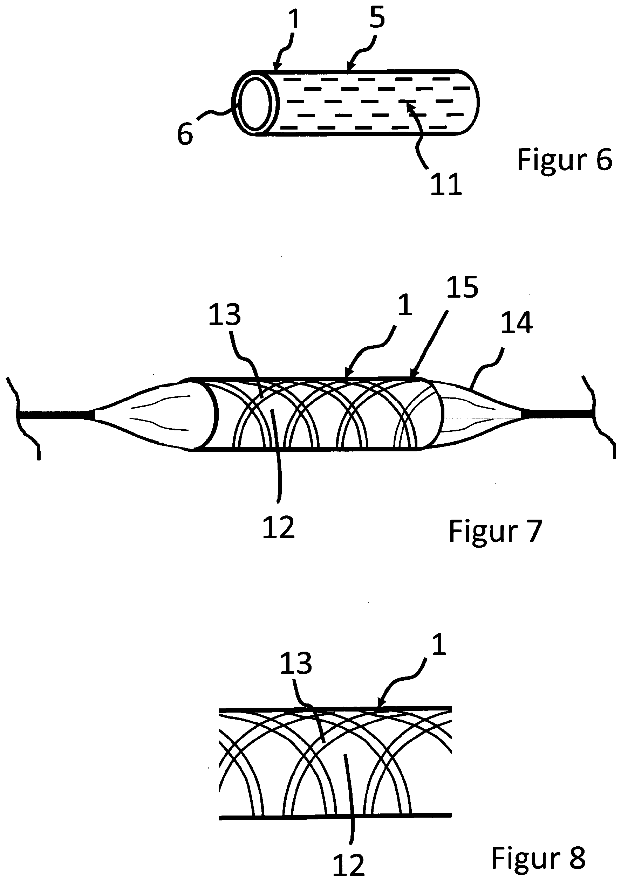

Zudem kann die Zwischenwandung 10 radial in etwa gleich beabstandet voneinander und über den gesamten Umfang verteilt in Hülsenlängsrichtung 2 Sollrissstellen 11 (präformierte Kerben, Sollbruchstellen, Verdünnungen) aufweisen (Figur 6), die ein definiertes bzw. gerichtetes Aufreißen der Zwischenwandung 10 ermöglichen. Vorzugsweise weisen diese Sollrissstellen 11 eine Länge von 200 µm bis 1000 µm auf.

Beim Entfalten und Entplissieren einer derartigen rohrförmigen Hülse 1 ist vorgesehen, dass die Zwischenwandung 10 entlang der Sollrissstellen bzw. der perforierten Kerben reißt. Das Reißen wird zudem dadurch begünstigt, dass bei einem Zylinder unter Innendruck Risse in Längsrichtung entstehen, da die tangentiale Wandspannung (quer zur Längsrichtung) in der Zwischenwandung 10 doppelt so hoch ist wie in Längsrichtung 2.

Auf diese Weise wird eine Verbindung zwischen dem inneren Kompartiment 8 und dem äußeren Kompartiment 9 derart bereitgestellt, dass die beiden darin enthaltenen Substanzen sich miteinander vermischen. Das im inneren Kompartiment 8 angeordnete Vernetzungsmittel bzw. das darin angeordnete Bindemittel kann z. B. auf Cyanakrylatbasis, wie N-Butyl-2-Cyanoakrylat, bestehen. Im äußeren Kompartiment 9 ist vorzugsweise Polylactid (PLA) als Nanopulver oder als Micro-Nano-Fasern angeordnet. Durch Kontaktieren des Vernetzungsmittels mit dem pulver- oder faserförmigen PLA findet eine Polymerisationsreaktion statt, die zu einer mechanischen Verfestigung des Hülseninhalts führt. Diese mechanische Verfestigung der entfalteten Hülse 1 verhindert ein Rekollabieren in den gefalteten plissierten Zustand.

Zudem kann vorgesehen sein, den Zusammenhalt der Außenwandung 5, der Zwischenwandung 10 und der Innenwandung 6 während des Plissier-, des Faltungs-, des Entfaltungs- und des Entplissierungsvorgangs durch Verbindungspunkte bzw. Verbindungslinien zwischen den Wandungen 5, 10, 6, die z.B. durch Laserschweißen erzeugt wurden, zu verstärken.

Gemäß einer weiteren Ausführungsform der vorliegenden Erfindung, die, soweit nichts anderes beschrieben ist, im Wesentlichen der zweiten Ausführungsform entspricht, kann die Hülse 1 eine Außenwandung 5 und eine Innenwandung 6 umfassen (Figuren 7 und 8).

Hierbei ist vorgesehen, dass die Außenwandung 5 und die Innenwandung 6 quer bzw. schräg zur Längsrichtung miteinander derart, z. B. mittels Laserschweißen, verbunden sind, dass entlang der gesamten Wandung der Hülse 1, bestehend aus Außenwandung 5 und Innenwandung 6, spiralförmig auf dem Umfang verlaufende Kanäle 13, ähnlich einem Rautenmuster, ausgebildet sind. Alternativ zum Laserschweißverfahren ist zum Erzeugen der Kanäle 13 auch ein Heißprägeverfahren auf einem Metallkern denkbar, der das gewünschte Streben- bzw. Kanalmuster durch entsprechende Vorsprünge erzeugt.

Die spiralförmigen Hohlräume bzw. Kanäle 13 können mit einer Trockensubstanz, wie z. B. einem Polylactidnanopulver, gefüllt sein, wobei die Zwischenräume 12 zwischen den Streben mit einem Vernetzungs- oder Bindemittel, z. B. auf Cyanacrylatbasis, versehen sind. Die Kanäle 13 und die Zwischenräume 12 bilden im Sinne der vorliegenden Erfindung Unter-Kompartimente aus.

Sowohl derartige Kanäle 13 als auch die Zwischenräume 12 stellen Kompartimente im Sinne der vorliegenden Erfindung dar.

Beim Entfalten und Entplissieren der rohrförmigen Hülse 1 bewirkt der beim Expandieren des Ballonkatheters 14 entstehende Druck, dass die mit flüssigem Vernetzungsmittel gefüllten Zwischenräume 12 zwischen den Kanälen 13 flachgepresst werden und das Vernetzungsmittel mechanisch auf die Kanäle 13 drückt, bis die Wandungen der Kanäle 13 reißen. Auf diese Weise vermischen sich das Bindemittel und das Nanopulver.

Die Folien, aus denen die Innenwandung 6 und die Außenwandung 5 ausgebildet sind, halten einem Ballonkatheterdruck bis ca. 25 atm Stand, ohne zu zerreißen. Die Schweißungen der Kanäle 13 haben eine sehr geringe mechanische Festigkeit. Im Ausgangszustand ist ein Plissieren und Falten sowie das Aufbringen auf einen Ballonkatheter derart möglich, dass die Kompartimente sicher voneinander getrennt sind. Jedoch bei geringer Druckbeaufschlagung durch den Ballon reißen diese auf, während die innere und die äußere Folie intakt bleiben.

Bei allen vorstehend beschriebenen Ausführungsformen, die sowohl eine flüssige Komponente in Form eines Binde- bzw. Vernetzungsmittels aufweisen, als auch eine feste Komponente in Form eines Pulvers oder von Fasern, ist es grundsätzlich auch möglich, die flüssige Komponente von außen, z. B. durch Beschickung über einen zusätzlichen Kanal eines Ballonkatheters, der eine komplette Durchflutung des Kompartiments erlaubt, zuzuführen. Gegebenenfalls kann auch ein Überlauf vorgesehen sein, der es erlaubt, überschüssige Flüssigkeit aus dem entsprechenden Kompartiment abzuführen.

Alternativ kann die für den Quellvorgang benötigte Flüssigkeit auch durch einen Kontakt mit Blutplasma bereitgestellt werden, wobei der Kontakt mit Blut über eine poröse bzw. permeable Außenwandung bzw. Membran mit entsprechender Porosität bzw. eine Vliesstruktur erfolgt. Hierfür gilt die eingangs erläuterte Definition der geschlossen ausgebildeten Mantelwandung.

Die Verfestigung der erfindungsgemäßen rohrförmigen Hülse 1 kann dadurch erfolgen, dass die beiden vorstehend beschriebenen Komponenten, wie z. B. Pulver oder Fasern, und das Binde- bzw. Vernetzungsmittel mechanisch zusammengeführt werden, d. h. durch Penetration bzw. Reißen von Zwischenwandungen zwischen Kompartimenten bzw. Unterkompartimenten. Weiterhin ist auch eine aktive Injektion oder ein passives Aufsaugen eines flüssigen Vernetzungsmittels möglich.

Gemäß einer weiteren Ausgestaltung des passives Aufsaugens eines flüssigen Vernetzungsmittels kann dieses Vernetzungsmittel auch Blutplasma sein, wobei das mikroporöse, mikrokompartimentierte Hülsenmaterial bevorzugt ein Vlies aus Polylactid-Fasern ist, das mit CalciumcloridSalz dotiert ist, um eine Fibrin-Polymerisationreaktion auszulösen.

Denkbar ist auch eine mechanische und thermische Einwirkung von Ultraschall. Hierbei ist es z. B. möglich, eine Ultraschallsonde zusammen mit dem Ballonkatheter zum Implantationsort einzubringen.

Alternativ ist auch eine optisch-thermische Zusammenführung durch Integration einer Lasereinrichtung in den Ballonkatheter und Einwirkung von UV-Licht, z. B. durch die Lasersinterung von PLA-Pulver, denkbar.

Weiterhin kann auch eine Hochfrequenz-Sondeneinrichtung vorgesehen sein, die ebenfalls im Ballonkatheter angeordnet ist und mittels thermischer Energie auf die rohrförmige Hülse einwirkt.

Auch eine chemische Stabilisierung durch sogenannte Klick-Chemie, wie der Thiol-En-Reaktion, z. B. durch Photopolymerisation und Integration einer UV-Lasereinrichtung in den Ballonkatheter ist möglich.

Alle vorstehend beschriebenen Ausführungsformen können vorsehen, dass auf der Außenwandung 5 der rohrförmigen Hülse 1 ein medizinischer Wirkstoff aufgebracht bzw. die Außenwandung 5 mit einem derartigen medizinischen Wirkstoff dotiert ist. Unter einem medizinischen Wirkstoff im Rahmen der vorliegenden Erfindung wird ein Antiproliferativum und/oder eine heilungsfördernde Substanz wie Fibringel verstanden. Derartige medizinische Wirkstoffe können auch mit autologen Zellen des Patienten vermischt sein, wobei ein Blutkontakt und eine Verhinderung von Thrombusbildung währen des Transportes der Hülse zum Implantationsort durch eine äußere Schutzhülle erfolgen kann. Eine derartige äußere Schutzhülle kann am Implantationsort durch Zurückziehen derselben erfolgen. Alternativ kann auch ein äußeres Sicherheitsnetz bzw. eine Sicherheitshülse vorgesehen sein, die beim Entfalten und Entplissieren zerreißt, da sie einen geringeren Entfaltungsdurchmesser als die rohrförmige Hülse 1 aufweist.

Die Stabilität der Faltung und der Plissur der Hülse 1 beim Transport bzw. beim Einbringen in das Hohlorgan kann durch eine adhäsive Oberflächenbehandlung der Außenwandung der Hülse unterstützt werden.

Die Innenwandung 6 kann innenseitig zudem mit einer antithrombogenen Substanz, wie z. B. Heparin, und/oder einem Wirkstoff dotiert sein, der die Endothelzellen-Besiedelung fördert.

Weiterhin kann unmittelbar vor der Implantation eine Vorbereitung bzw. ein Priming der Innen-und Außenwandungen der Hülse erfolgen, indem die Oberfläche mit körpereigenen Substanzen des Patienten, wie z. B. Vollblut, Plasma, Serum oder Zellen benetzt wird.

Zudem kann eine Benetzung der Innen- und Außenwandung der Hülse während des Implantati-onsvorgans in situ mit bioaktiven Wirkstoffen oder körpereigenen Substanzen des Empfängerpatienten erfolgen, indem diese über Beschickungskanäle eingebracht werden, wobei diese Teil des Ballonkatheters sind und mit der rohrförmigen Hülse 1 derart verbunden sind, dass sie zur Innen- bzw. zur Außenseite der Hülse führen.

Die Wandungen bzw. deren Eigenschaften zur Haftung und Speicherung von Wirkstoffen kann durch eine Oberflächenstrukturierung, eine Gelfilmauflagerung oder entsprechende Poren verbessert werden.

Gemäß einem erfindungsgemäßen Verfahren zur Herstellung eines Systems zur atraumatischen Behandlung von Hohlorganen, ist vorgesehen, dass eine Ballonfolie eines Ballonkatheters zusammen mit der außen koaxial daran anliegenden rohrförmigen Hülse gemeinsam in einem Arbeitsschritt plissiert und gefaltet werden.

Im Folgenden werden verschiedene Ausführungsformen einer Mantelwandung 15 einer erfindungsgemäßen Hülse 1 beschrieben.

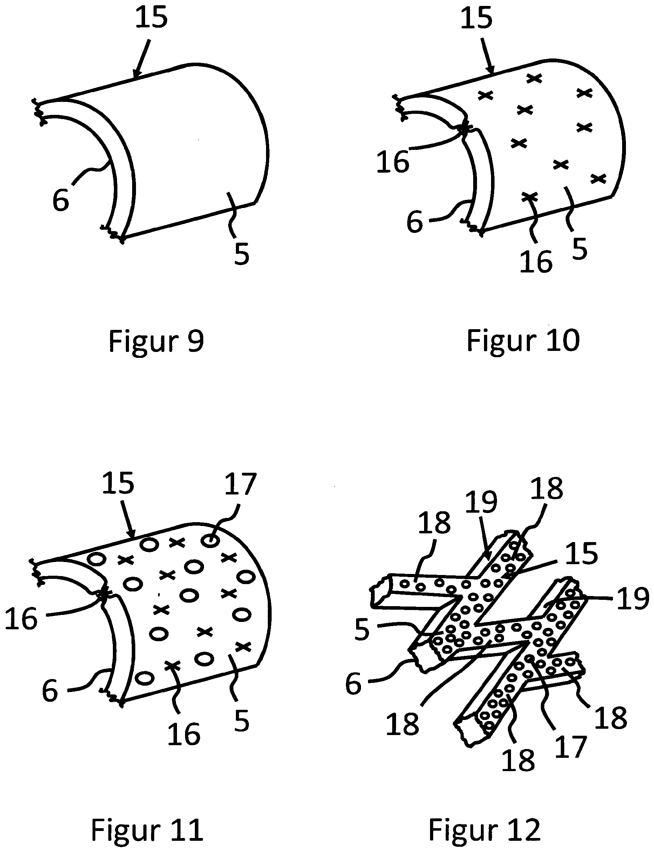

Zwischen Innen- und Außenwandung 6, 5 können Verbindungspunkte 16 ausgebildet sein, die in etwa gleichmäßig über die gesamte Mantelwandung 15 verteilt angeordnet sind (Figur 10).

Vorzugsweise sind die Innen- und die Außenwandung 6, 5 im Bereich der Verbindungspunkte 16 direkt derart miteinander verbunden, dass im Bereich der Verbindungspunkte Innen- und Außenwandung 6, 5 einander kontaktieren. Die Verbindungspunkte können z. B. durch Schweiß- oder Klebeverbindungen realisiert sein. Im Rahmen der vorliegenden Erfindung können die Verbindungspunkte auch als sich zwischen Innen- und Außenwandung erstreckende Stege (nicht dargestellt), die vorzugsweise aus demselben Material wie die Hülse ausgebildet sind, vorgesehen sein.

An Stelle oder zusätzlich zu den Verbindungspunkten 16 können auch durchgehende oder abschnittsweise ausgebildete Verbindungslinien (nicht dargestellt) vorgesehen sein.

Gemäß einem weiteren Ausführungsbeispiel der Mantelwandung 15 sind neben den Verbindungspunkten 16 auch etwa gleichmäßig über die Außenwandung der Mantelwandung verteilt angeordnete Öffnungen bzw. Poren 17 vorgesehen (Figur 11). Die in der Hülse ausgebildeten Poren 17 einen Durchmesser im Bereich zwischen 1 µm und 100 µm, bzw. zwischen 5 µm und 80 µm, bzw. zwischen 5 µm und 60 µm, bzw. zwischen 5 µm und 40 µm, bzw. zwischen 5 µm und 30 µm bzw. zwischen 5 µm und 10 µm und vorzugsweise zwischen 5 µm und 20 µm aufweisen.

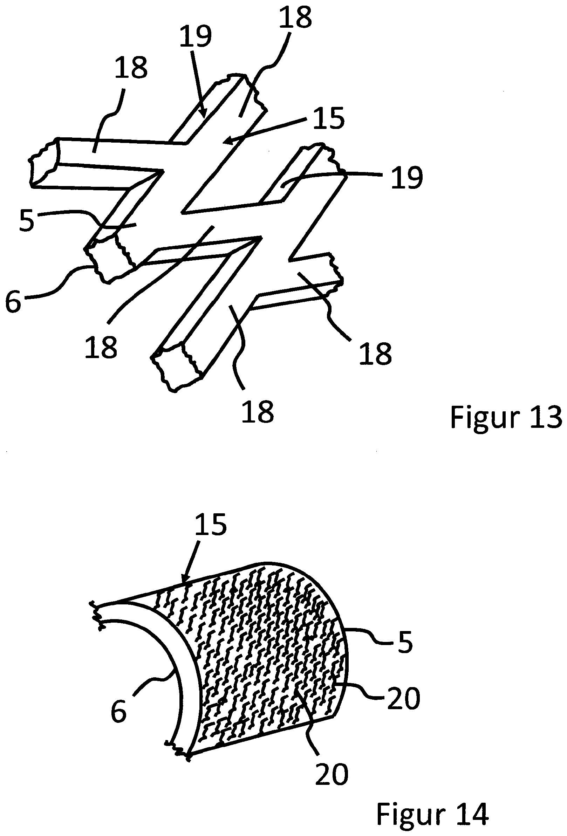

Gemäß einem weiteren Ausführungsbeispiel kann die gesamte Mantelwandung 15 als Gitterstruktur ausgebildet sein (Figuren 12 und 13). Die einzelnen Streben 18 der Gitterstruktur, insbesondere die Außen-, Innen- und auch ggf. vorhandene Seitenwandungen 19, begrenzen dann das Kompartiment der Hülse.

Bei diesem Ausführungsbeispiel können die Gitterstreben 18 ebenfalls die bereits vorstehend beschriebenen Öffnungen, Verbindungspunkte, Trennwandungen oder Stege derart aufweisen, dass in der Gitterstruktur mehrere Kompartimente 7 ausgebildet sind.

Gemäß einem weiteren Ausführungsbeispiel weist die Außenwandung der Mantelwandung 15 sich in Richtung des Hohlorgans erstreckende Zapfen 20 bzw. Mikro-Fibrillen auf, die eine Mikrostruktur ausbilden (Figur 14). Eine derartige Mikrostruktur ist insbesondere zur Bindung eines Hydrogels und/oder eines medizinischen Wirkstoffs geeignet.

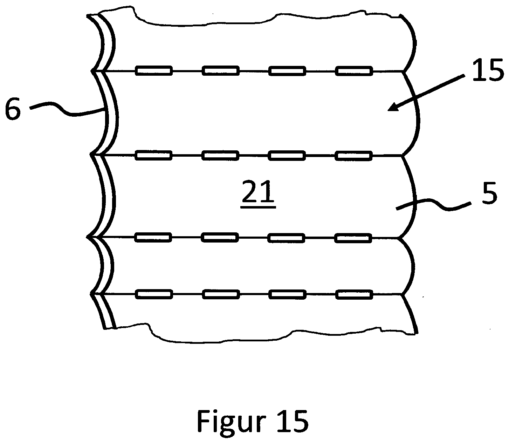

Gemäß einem weiteren Ausführungsbeispiel ist die Mantelwandung aus sich in Längsrichtung erstreckenden Mantelwandungsabschnitten ausgebildet, die quer zur Längsrichtung bzw. in radialer Richtung gelenkartig miteinander verbunden sind (Figur 15). Derartige Mantelwandungsabschnitte 21 weisen, wie die vorstehend beschriebenen Ausführungsformen, entsprechende Kompartimente 7 auf.

Gemäß einem alternativen Ausführungsbeispiel der vorliegenden Erfindung ist die gesamte rohrförmige Hülse aus einem Vliesstoff ausgebildet (nicht dargestellt). Demgemäß wird sowohl die Außen- als auch die Innenwandung 5, 6 durch den Vliesstoff ausgebildet. Auch im Bereich zwischen Außen- und Innenwandung 5, 6 ist ein Vliesstoff vorgesehen. Demgemäß kann die gesamte Mantelwandung 15 der rohrförmigen Hülse als integrale Vliesstoffstruktur ausgebildet sein. Die Kompartimente 7 sind dann als Mikrokompartimente im Bereich zwischen den einzelnen Fasern des Vliesstoffes vorgesehen. In diesen Mikrokompartimenten 7 kann ebenfalls ein Füllstoff angeordnet, eingelagert oder gebunden sein.

Nachfolgend werden weitere Vorteile einzelner Ausgestaltungen sowie beispielhaft vorteilhafte Ausführungsformen beschrieben.

Sämtliche Ausführungsformen der vorliegenden Erfindung können in der Innen- und/oder auch in der Außenwandung 6, 5 Poren 17 aufweisen.

Sämtliche Ausführungsformen der vorliegenden Erfindung können auf der Innen- und/oder der Außenwandung 6, 5 Zapfen aufweisen, die sich dann in Richtung einer Mittelachse der Vorrichtung oder entsprechend radial nach außen erstrecken.

Sämtliche Ausführungsformen der vorliegenden Erfindung können zwischen der Innen- und der Außenwandung 6, 5 und/oder zwischen diesen und einer oder mehreren koaxialen oder radialen Zwischenwandungen Stege, Verbindungs-linien, -punkte, Wandungen bzw. Wandungsabschnitte aufweisen.

Das Kompartiment 7 bzw. die Kompartimente ermöglichen eine gute räumliche Ausgestaltung für ein Wirkstoffdepot. Ein solches Wirkstoffdepot ermöglicht bspw. eine Einlagerung eines wirkstoffdotierten Gels.

Ein bevorzugtes Ausführungsbeispiel, umfasst eine plissierte Hülse (als Folie) aus PLA mit einer Wandstärke im Bereich zwischen 20-50 µm, wobei die äußere Mantelfläche mit einem Hydrogel und/oder einem Kleber beschichtet ist. Die innere Mantelfläche kann chemisch biofunktionalisiert oder mit einem Wirkstoff beschichtet sein.

Ein weiteres bevorzugtes Ausführungsbeispiel, umfasst eine innere dicke Folie mit einer Wandstärke im Bereich zwischen 20-40 µm einer äußeren dünnen Folie mit einer Wandstärke im Bereich zwischen 5-10 µm, wobei letztere multiple Poren aufweist. Im Kompartiment zwischen beiden Folien befindet sich eine Schicht wirkstoffbeladenes Hydrogel, das so als Depot vor Ort gehalten wird, der Wirkstoff jedoch über die Poren auf der Außenfläche nach und nach abgegeben werden kann.

Ein derartiger Aufbau kann beliebig erweitert werden, bspw. mit einer weiteren porösen Folie als Zwischenwandung, mit einem zweiten, anderen Wirkstoff-Depot. Oder mit einer Unterteilung des röhren- bzw. hülsenförmigen Kompartiments in eine Vielzahl von Unterkompartimenten, aus denen ggfs. noch eine größere Anzahl an Mikro-Kompartimenten resultieren.

Weiterhin kann die die Innenwandung ausbildende Folie eine Dicke von z.B. 90 µm aufweisen. Auf diese Weise würde die Innenwandung einen Stentkörper mit ausreichender radialer Stützkraft ausbilden, der ein Gefäßlumen stützen kann, das bspw. nach einer Ballondilatation zu kollabieren droht. Zur Verbesserung der Faltung der relativ dicken Folie kann diese Längsschlitze aufweisen, die den Faltungsknicken entsprechen (Fig. 15).

Demgemäß können die Innen- und/oder Zwischen und/oder Außenwandung einer erfindungsgemäßen Hülse eine vorbestimmte radiale Stützkraft aufweisen, die dann die Radiale Stützkraft der Hülse bestimmt. Z.B. kann die Zwischenwandung relativ dick ausgebildet sein und in Kompartimenten zwischen zwischen Zwischenwandung und Außen und Innenwandung ist je ein wirkstoffgefülltes Kompartiment ausgebildet, so dass die Zwischenwandung die radiale Stützkraft bestimmt. Gleiches ist mit einer dickeren Außen- oder Innenwandung mit oder ohne Zwischenwandung möglich.

### Bezuaszeichenliste

- 1: rohrförmige Hülse
- 2: Hülsenlängsachse
- 3: Außenwandung
- 4: Hydrogelschicht
- 5: Außenwandung
- 6: Innenwandung
- 7: Kompartiment
- 8: inneres Kompartiment
- 9: äußeres Kompartiment
- 10: Zwischenwandung
- 11: Sollrissstellen
- 12: Zwischenräume
- 13: Kanäle
- 14: Ballonkatheter
- 15: Mantelwandung
- 16: Verbindungspunkt
- 17: Pore
- 18: Strebe
- 19: Seitenwandung
- 20: Zapfen
- 21: Mantelwandungsabschnitt

## Patentansprüche

1. Rohrförmige Hülse zur atraumatischen Behandlung von Hohlorganen, wobei die Hülse in einem Ausgangszustand gefaltet ist und in einem Endzustand zum Anliegen an einer Innenwandung eines Hohlorganes entfaltbar ist,
**dadurch gekennzeichnet,**
**dass** die Hülse aus einer Außenwandung und einer Innenwandung ausgebildet ist, die konzentrisch zueinander angeordnet sind, wobei die Außenwandung und die Innenwandung stirnseitig derart dicht miteinander verbunden sind, dass im Bereich zwischen der Außenwandung und der Innenwandung ein Kompartiment ausgebildet ist und die Faltung der Hülse als Plissierung um eine Hülsenlängsachse gerichtet ist, und wobei
sowohl eine Außenwandung als auch eine Innenwandung und ein in dem Kompartiment zwischen Außen- und Innenwandung angeordneter Füllstoff der Hülse aus einer textilen Struktur ausgebildet ist, wobei die textile Struktur aus Fäden gewoben, gewirkt, geflochten, gestrickt, geklöppelt oder aus einem Vlies, vorzugsweise aus ein und demselben Vlies oder aber auch aus zwei verschiedenen Vliesstoffen ausgebildet sind, wobei dann eine Außenfläche des Vlieses eine Außenwandung und eine Innenfläche des Vlieses die Innenwandung ausbildet und die Zwischenräume der Fasern im Kompartiment Mikrokompartimente ausbilden; und
eine Mantelwandung der Hülse adhäsive Eigenschaften aufweist und/oder mit einer Beschichtung derart versehen ist, dass die Mantelwandung beim Entfalten an einer Innenwandung des Hohlorganes anhaftet, und wobei
die Hülse mit einer bioaktiven Substanz dotiert ist.

2. Rohrförmige Hülse gemäß Anspruch 1,
**dadurch gekennzeichnet,**
**dass** die bioaktive Substanz ein medizinischer Wirkstoff ist, der ein Antiproliferativum ist.

3. Rohrförmige Hülse gemäß Anspruch 1 oder 2,
**dadurch gekennzeichnet,**
**dass** die Beschichtung eine Hydrogelschicht ist.

4. Rohrförmige Hülse gemäß einem der Ansprüche 1 bis 3,
**dadurch gekennzeichnet,**
**dass** die rohrförmige Hülse eine gitterartige Mantelwandung aufweist.

5. Rohrförmige Hülse gemäß einem der Ansprüche 1 bis 4,
dass die Mantelwandung der Hülse geschlossen ausgebildet ist, d.h. deren Innen- und/oder Außenwandung eine Porosität aufweist, wobei die in der Hülse ausgebildeten Poren einen maximalen Durchmesser kleiner 100 µm, bzw. kleiner 80 µm, bzw. kleiner 60 µm, bzw. kleiner 40 µm, bzw. kleiner 30 µm bzw. kleiner 10 µm und vorzugsweise kleiner 20 µm aufweisen.

6. Rohrförmige Hülse gemäß einem der Ansprüche 1 bis 5,
**dadurch gekennzeichnet,**
**dass** die Dicke der Wandungen in etwa 20 µm beträgt.

7. Rohrförmige Hülse gemäß einem der Ansprüche 1 bis 6,
**dadurch gekennzeichnet,**
**dass** die Hülse zylindrisch, konisch oder eingeschnürt ausgebildet ist.

8. Rohrförmige Hülse gemäß einem der Ansprüche 1 bis 7,
**dadurch gekennzeichnet,**
**dass** die Mantelwandung der Hülse, d.h. deren Innen- und/oder Außenwandung mit Zapfen versehen sind.

9. Rohrförmige Hülse nach einem der Ansprüche 1 bis 8,
**dadurch gekennzeichnet,**
**dass** die Mikrokompartimente mit einem Füllstoff oder mit einem flüssigen Vernetzungsmittel oder einem flüssigen Bindemittel gefüllt sind.

10. Rohrförmige Hülse nach einem der Ansprüche 1 bis 9,
**dadurch gekennzeichnet,**
**dass** die Hülse mit einer heilungsfördernden bioaktiven Substanz, wie z.B. Fibringel, versehen ist, wobei die Substanz optional mit autologen Zellen eines Patienten vermischt sind.

11. Rohrförmige Hülse gemäß einem der Ansprüche 1 bis 10,
**dadurch gekennzeichnet,**
**dass** in der Hülse im entfalteten Zustand eine in situ-Polymerisation mindestens zweier Komponenten in den Mikrokompartimenten stattfindet.

12. Rohrförmige Hülse gemäß Anspruch 11,
**dadurch gekennzeichnet,**
**dass** die Polymerisation bewirkt wird durch
ein passives Aufsaugen eines flüssigen Vernetzungsmittels, wobei dieses flüssige Vernetzungsmittel auch eine Körperflüssigkeit wie Blutplasma sein kann und die Hülse, bevorzugt als Vlies, mit Calciumcloridsalz dotiert ist, um eine Fibrinpolymersation in Gang zu setzen.

13. Rohrförmige Hülse gemäß einem der Ansprüche 1 bis 12,
**dadurch gekennzeichnet,**
**dass** die Dotierung einer Innenfläche der Hülse mit einer antithrombogenen Substanz, wie z.B. Heparin, und/oder einem Wirkstoff erfolgt, der eine Endothelzellen-Besiedlung fördert.

14. System zur atraumatischen Behandlung von Hohlorganen umfassend
einen Ballonkatheter und
eine rohrförmige Hülse nach einem der Ansprüche 1 bis 13.

15. Verfahren zur Herstellung eines Systems zur atraumatischen Behandlung von Hohlorganen,
**dadurch gekennzeichnet,**
**dass** eine Ballonfolie eines Ballonkatheters zusammen mit der außen koaxial daran anliegenden rohrförmigen Hülse gemäß einem der Ansprüche 1 bis 13 gemeinsam in einem Arbeitsschritt plissiert und gefaltet werden.

## Claims

1. A tubular sleeve for atraumatic treatment of hollow organs, wherein the sleeve is folded in an initial state, and deployable to attach to an inner wall of a hollow organ in an end state,
**characterized in that**
the sleeve is formed from an outer wall and an inner wall, which are arranged concentric to each other, wherein the outer wall and the inner wall are connected to one another on the end faces so tightly that in the region between the outer wall and the inner wall a compartment is formed, and the folding of the sleeve is carried out as a pleating around a longitudinal axis of the sleeve, and wherein
both an outer wall and an inner wall of the sleeve and a filler of the sleeve arranged in the compartment between the outer and inner wall is made of a textile structure, wherein the textile structure is woven, knitted, braided, lace-made from threads or is made of a nonwoven, preferably of one and the same nonwoven, or of two different nonwoven fabrics, wherein then an outer surface of the nonwoven forms an outer wall and an inner surface forms an inner wall and the interspaces between fibers in the compartment form micro-compartments, and
a circumferential wall of the sleeve has adhesive properties and/or is provided with a coating so that the circumferential wall adheres to an inner wall of the hollow organ during deployment,
and
the sleeve is doped with a bioactive substance.

2. A tubular sleeve according to claim 1,
**characterized in that**
the bioactive substance is a medical compound which is an antiproliferative.

3. A tubular sleeve according to claim 1 o 2,
**characterized in that**
the coating is a layer of hydrogel.

4. A tubular sleeve according to one of the claims 1 to 3,
**characterized in that**
the tubular sleeve has a lattice-like circumferential wall.

5. A tubular sleeve according to one of the claims 1 to 4,
**characterized in that**
circumferential wall of the tubular sleeve is AUSGEBILDET, closed, namely in a manner that its inner and/or outer wall has a porosity, wherein the pores formed in the sleeve have a maximum diameter of less than 100 µm, or less than 80 µm, or less than 60 µm, or less than 40 µm, or less than 30 µm or less than 10 µm and preferably less than 20 µm.

6. A tubular sleeve according to one of the claims 1 to 5,
**characterized in that**
the thicknesses of the walls amount to approximately 20 µm.

7. A tubular sleeve according to one of the claims 1 to 6,
**characterized in that**
the sleeve is cylindrical, conical or constricted.

8. A tubular sleeve according to one of claims 1 to 7,
**characterized in that**
the circumferential wall of the sleeve, that is to say the inner and/or outer wall surface are provided with pins.

9. A tubular sleeve according to one of the claims 1 to 8,
**characterized in that**
the microcompartments are filled with a filler or a liquid cross-linking agent or a liquid binding agent.

10. A tubular sleeve according to one of the claims 1 t 9,
**characterized in that**
the sleeve is provided with a healing-promoting bioactive substance, such as, e.g. fibrin gel, wherein the substance is optionally mixed with autologous cells of a patient.

11. A tubular sleeve according to one of the claims 1 to 10,
**characterized in that**
in the sleeve, in the deployed state, takes place in situ polymerization of at least two components in the micro-compartments.

12. A tubular sleeve according to claim 11,
**characterized in that**
the polymerization is caused by a passive absorption of a liquid crosslinking agent, wherein this crosslinking agent can be a bodily fluid like blood plasma, and the sleeve, preferably as a nonwoven, is doped with calcium chloride salt to initiate fibrin polymerization.

13. A tubular sleeve according to one of the claims 1 to 12,
**characterized in that**
a doping of an inner surface of the sleeve is carried out with an antithrombogenic substance such as, for example heparin, and / or an active substance, which promotes endothelial cell coating.

14. System for the atraumatic treatment of hollow organs
comprising
a balloon catheter
and the tubular sleeve according to one of the claims 1 to 13.

15. Method for the production of a system for the atraumatic treatment of hollow organs **characterized in that**
a balloon foil of a balloon catheter together with the tubular sleeve according to one of the claims 1 to 13 coaxially fitted closely to the outside of the same are folded and pleated together in one process step.

## Revendications

1. Douille tubulaire servant au traitement atraumatique d'organes creux, dans laquelle la douille est pliée dans un état de départ et peut être dépliée dans un état final pour reposer au niveau d'une paroi intérieure d'un organe creux,
**caractérisée en ce**
**que** la douille est réalisée à partir d'une paroi extérieure et d'une paroi intérieure, qui sont disposées de manière concentrique l'une par rapport à l'autre, dans laquelle la paroi extérieure et la paroi intérieure sont reliées l'une à l'autre côté frontal de telle manière étanche qu'un compartiment est réalisé dans la zone entre la paroi extérieure et la paroi intérieure et le pliage de la douille sous la forme d'un plissage est dirigé autour de l'axe longitudinal de douille, et dans laquelle
à la fois la paroi extérieure et la paroi intérieure et une charge, disposée dans le compartiment entre la paroi extérieure et la paroi intérieure, de la douille sont réalisées à partir d'une structure textile, dans laquelle la structure textile est tissée, tressée, entrelacée, tricotée, maillée à partir de fils ou est réalisée à partir d'un non-tissé, de préférence à partir d'un seul et même non-tissé ou toutefois également à partir de différents tissus non-tissés, dans laquelle alors une face extérieure du non-tissé réalise une paroi extérieure et une face intérieure du non-tissé réalise la paroi intérieure et les espaces intermédiaires des fibres réalisent dans le compartiment des microcompartiments ; et
une paroi enveloppante de la douille présente des propriétés adhésives et/ou est pourvue d'un revêtement de telle manière que la paroi enveloppante adhère lors du dépliage contre une paroi intérieure de l'organe creux, et dans laquelle la douille est dopée avec une substance bioactive.

2. Douille tubulaire selon la revendication 1,
**caractérisée en ce**
**que** la substance bioactive est un principe actif médical, qui est un antiprolifératif.

3. Douille tubulaire selon la revendication 1 ou 2,
**caractérisée en ce**
**que** le revêtement est une couche d'hydrogel.

4. Douille tubulaire selon la revendication 1 ou 2,
**caractérisée en ce**
**que** la douille tubulaire présente une paroi enveloppante de type grille.

5. Douille tubulaire selon l'une quelconque des revendications 1 à 4,
**caractérisée en ce**
**que** la paroi enveloppante de la douille est réalisée de manière fermée, en d'autres termes sa paroi intérieure et/ou extérieure présente une porosité, dans laquelle les pores réalisés dans la douille présentent un diamètre maximal inférieur à 100 µm ou inférieur à 80 µm ou inférieur à 60 µm ou inférieur à 40 µm ou inférieur à 30 µm ou inférieur à 10 µm et de préférence inférieur à 20 µm.

6. Douille tubulaire selon l'une quelconque des revendications 1 à 5,
**caractérisée en ce**
**que** l'épaisseur des parois est d'environ 20 µm.

7. Douille tubulaire selon l'une quelconque des revendications 1 à 6,
**caractérisée en ce**
**que** la douille est réalisée de manière cylindrique, conique ou de manière étranglée.

8. Douille tubulaire selon l'une quelconque des revendications 1 à 7,
**caractérisée en ce**
**que** la paroi enveloppante de la douille, en d'autres termes sa paroi intérieure et/ou extérieure, sont pourvues de touillons.

9. Douille tubulaire selon l'une quelconque des revendications 1 à 8,
**caractérisée en ce**
**que** les microcompartiments sont remplis d'une charge ou d'un agent de réticulation liquide ou d'un liant liquide.

10. Douille tubulaire selon l'une quelconque des revendications 1 à 9,
**caractérisée en ce**
**que** la douille est pourvue d'une substance bioactive favorisant la guérison, telle que Fibringel, dans laquelle la substance est mélangée en option à des cellules autologues d'un patient.

11. Douille tubulaire selon l'une quelconque des revendications 1 à 10,
**caractérisée en ce**
**que** dans l'état déplié de la douille une polymérisation in situ d'au moins deux composants a lieu dans les microcompartiments.

12. Douille tubulaire selon la revendication 11,
**caractérisée en ce**
**que** la polymérisation est entraînée par
une aspiration passive d'un agent de réticulation liquide, dans laquelle ledit agent de réticulation passif peut être également un liquide corporel tel que du plasma sanguin, et la douille, de préférence sous la forme d'un non-tissé, est dopée de sel de chlorure de sodium afin de mettre en œuvre une polymérisation de fibrine.

13. Douille tubulaire selon l'une quelconque des revendications 1 à 12,
**caractérisée en ce**
**que** le dopage d'une face intérieure de la douille est effectué avec une substance antithrombogène, telle que de l'héparine, et/ou un principe actif, qui favorise une colonisation de cellules endothéliales.

14. Système de traitement atraumatique d'organes creux comprenant
un cathéter à ballonnet et
une douille tubulaire selon l'une quelconque des revendications 1 à 13.

15. Procédé de fabrication d'un système de traitement atraumatique d'organes creux, **caractérisé en ce**
**qu'**un film de ballonnet d'un cathéter à ballonnet est plissé et plié lors d'une étape de travail conjointement avec la douille tubulaire selon l'une quelconque des revendications 1 à 13 située au niveau de ce dernier de manière coaxiale.
